(19) Europäisches Patentamt
European Patent Office
Office européen des brevets

(11) **EP 3 780 196 A1**

(12) **EUROPEAN PATENT APPLICATION**
published in accordance with Art. 153(4) EPC

(43) Date of publication:
**17.02.2021 Bulletin 2021/07**

(51) Int Cl.:
*H01M 4/88* (2006.01)    *B01J 35/10* (2006.01)
*C01B 32/00* (2017.01)   *G06F 17/50* (2006.01)
*G16Z 99/00* (2019.01)   *H01M 4/86* (2006.01)
*H01M 4/96* (2006.01)    *H01M 8/10* (2016.01)

(21) Application number: 19785874.9

(22) Date of filing: 20.03.2019

(86) International application number:
**PCT/JP2019/011625**

(87) International publication number:
**WO 2019/198447 (17.10.2019 Gazette 2019/42)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **12.04.2018   JP 2018076523**

(71) Applicant: **Panasonic Intellectual Property
Management Co., Ltd.
Osaka-shi, Osaka 540-6207 (JP)**

(72) Inventors:
• **FUJITA Yusuke**
**Osaka-shi, Osaka 540-6207 (JP)**
• **YAMAMOTO Keiichi**
**Osaka-shi, Osaka 540-6207 (JP)**
• **MATSUSHITA Fumiya**
**Osaka-shi, Osaka 540-6207 (JP)**

(74) Representative: **Grünecker Patent- und
Rechtsanwälte
PartG mbB
Leopoldstraße 4
80802 München (DE)**

(54) **PARAMETER DETERMINATION METHOD AND SIMULATION METHOD FOR FINDING TRANSPORTABILITY OF GAS OR IONS WITHIN PORE**

(57)    The parameter determination method according to the present disclosure is a parameter determination method for determining a value of a parameter that is used for a simulation of determining gas transportability in a space inside a pore and that defines a boundary condition at an interface between a wall surface and gas or ions inside the pore, the method including determining the value of a parameter that reproduces a first concentration ratio indicating a ratio of the gas or ion concentration inside the pore to the gas or ion concentration outside the pore as the value of the parameter that defines the boundary.

FIG. 6

```
          START
            |
  CONSTRUCTION OF MODEL        ~ S61
            |
  MOLECULAR DYNAMICS CALCULATION ~ S62
            |
  CALCULATION OF DIFFUSION EQUATION  ~ S63
            |
     NO CHANGE IN GAS      ~ S64
      CONCENTRATION  --- NO --->
            |                  |
           YES                 |
            |        RESETTING OF ADSORPTION  ~ S66
     CONCORDANCE              PARAMETER
    BETWEEN GAS     ~ S65
    CONCENTRATION  --- NO --->
       RATIOS
            |
           YES
            |
           END
```

EP 3 780 196 A1

## Description

### Technical Field

The present disclosure relates to a simulation of determining gas or ion transportability inside a pore with high accuracy. In particular, the present disclosure relates to a parameter determination method for determining the value of a parameter that is applied to a simulation of determining gas or ion transportability inside a pore with high accuracy and relates to a simulation method for determining gas or ion transportability inside a pore by applying the parameter value determined by the parameter determination method.

### Background Art

In accordance with an expansion of the Ene-Farm market and the like, to date, research on improvement of performance and cost reduction of fuel cells has been performed. Under such circumstances, it is important to control a catalyst layer serving as the core for a stack or MEA (membrane electrode assembly) that is the heart of a fuel cell system, and ultimately, it is desirable that the optimum structure of the catalyst layer based on the operation conditions and the cell structure and the optimum production process of the catalyst layer can be determined without performing a trial production. In other words, proposal of a simulation technique for designing an optimum catalyst layer has been highly desired.

In general, a catalyst layer has a porous structure and is composed of a metal catalyst, a carbon carrier that carries the metal catalyst and that conducts electrons, a polymer electrolyte that conducts protons to the metal catalysts, and pores that diffuse gases, for example, hydrogen and oxygen.

However, in the above-described configuration, there is a problem in that trade-off occurs regarding the power generation performance of the fuel cell, where direct contact of the polymer electrolyte responsible for proton conduction with the metal catalyst ensures proton transportability but catalytic activity is degraded because of the metal catalyst being poisoned by the polymer electrolyte.

Accordingly, a simulation method in which a configuration exploiting liquid water instead of the polymer electrolyte as proton feed paths to the metal catalyst is assumed, material transport and electrochemical characteristics in the liquid water inside pores are calculated, and power generation performance inside pores is predicted has been proposed (for example, NPL 1).

### Citation List

### Non Patent Literature

NPL 1: T. Muzaffar, T. Kadyk, and M. Eikerling, "Physical Modeling of the Proton Density in Nanopores of PEM Fuel Cell Catalyst Layers", Electrochimica Acta 245 (2017) p. 1048-1058

### Summary of Invention

### Technical Problem

It is an issue of the present disclosure to provide a parameter determination method for determining the value of a parameter that is used for, for example, a simulation technique for determining gas or ion transportability inside a pore with high accuracy and to provide a simulation method for determining gas or ion transportability inside a pore.

### Solution to Problem

To address the above-described issue, a parameter determination method according to an aspect of the present disclosure is a parameter determination method for determining a value of a parameter that is used for a simulation of determining gas or ion transportability in a space inside a pore and that defines a boundary condition at an interface between a wall surface and gas or ions inside the pore, the method including determining the value of a parameter that reproduces a first concentration ratio indicating a ratio of the gas or ion concentration inside the pore to the gas or ion concentration outside the pore as the value of the parameter that defines the boundary.

To address the above-described issue, a simulation method according to an aspect of the present disclosure is a simulation method for determining gas or ion transportability inside a pore, the method including calculating a change in the gas or ion concentration on the basis of a diffusion equation in which the parameter value determined by the parameter determination method including determining the value of a parameter that reproduces a first concentration ratio indicating the ratio of the gas or ion concentration inside the pore to the gas or ion concentration outside the pore as the value of the parameter that defines a boundary condition at the interface between a wall surface and gas or ions in the space inside the pore is applied as the value of the parameter that defines the boundary condition at the interface between the wall surface and the gas or ions inside the pore.

### Advantageous Effects of Invention

The present disclosure includes the above-described steps and has an effect of enabling the value of the parameter that is used for a simulation technique for calculating gas or ion transportability inside a pore with high accuracy to be determined.

### Brief Description of Drawings

[Fig. 1] Fig. 1 is a schematic diagram showing an example of a carbon carrier having a pore according to an embodiment of the present disclosure.

[Fig. 2] Fig. 2 is a diagram showing an example of each graphite face constituting a model simulating a space inside pore of a carbon pore in a carbon carrier and a space outside pore of the carbon pore according to an embodiment of the present disclosure.

[Fig. 3] Fig. 3 is a diagram showing an example of a model simulating a space inside pore of a carbon pore formed by using the graphite faces shown in Fig. 2 and a space outside pore of the carbon pore.

[Fig. 4] Fig. 4 is a conceptual diagram showing an example of a method for determining an adsorption parameter of oxygen with respect to a wall surface according to an embodiment of the present disclosure.

[Fig. 5] Fig. 5 is a schematic diagram showing an example of a model used to calculate an oxygen concentration according to an embodiment of the present disclosure.

[Fig. 6] Fig. 6 is a flow chart showing an example of a method for determining an adsorption parameter according to an embodiment of the present disclosure.

Description of Embodiments

(Underlying knowledge forming basis of embodiment according to present disclosure)

The present inventors performed intensive research on the above-described trade-off problem related to power generation performance and, as a result, found the following.

In this regard, the following configuration may be proposed as the configuration that addresses the above-described trade-off problem. That is, in the configuration, the property that a polymer electrolyte cannot enter a small pore is exploited, the metal catalysts are carried inside the pore included in the carbon carrier, and the liquid water inside the pore rather than the polymer electrolyte is used as proton feed paths to the metal catalysts. Adopting such a configuration enables direct contact between the polymer electrolyte and the metal catalysts to be suppressed and degradation in the activity of the metal catalysts to be suppressed.

However, there are many unclear points regarding physical phenomena in the liquid water inside the pore. Therefore, it is necessary to develop a new simulation technique to quantitatively predict the pore condition most suitable for improving the power generation performance. In particular, it is important to develop a simulation technique to quantitatively predict the material transportability inside the pore that relates to the power generation performance to a great extent. For example, NPL 1 above proposes a simulation technique to calculate material transport and electrochemical characteristics inside the pore when the liquid water is used as the proton feed paths to the metal catalysts. According to NPL 1, the power generation performance inside the pore is predicted by this simulation technique.

In this regard, the present inventors particularly noted the transportability of gas, for example, oxygen, among the material transportability inside a nanoscale pore. As a result, it was found that, when the gas transportability inside the pore was evaluated, an aspect of gas adsorption and desorption with respect to the wall surface inside the pore had to be taken into consideration.

That is, the present inventors verified by the molecular dynamics calculation that the gas concentration inside the pore became higher than the gas concentration in a gas phase space outside the pore due to adsorption. From this result, it was found that the gas inside the pore was transported while repeating adsorption to and desorption from the wall surface. Consequently, it was found that, when the gas transportability inside the pore was evaluated, an aspect in which the gas is transported while repeating adsorption to and desorption from the wall surface had to be taken into consideration.

However, in NPL 1, the above-described aspect of the gas inside the pore (gas adsorption to and desorption from the pore wall surface) in the system in which circular cylindrical metal catalysts are present inside the pore formed of the polymer electrolyte is not taken into consideration.

More specifically, it is considered that trade-off occurs inside the pore where transport of the gas is hindered by adsorption of the gas to the pore wall surface whereas the reactivity of the catalyst is improved because of an increase in the gas concentration inside the pore. However, the present inventors found a problem in that, in NPL 1, gas adsorption to and desorption from the pore wall surface was not taken into consideration and the gas transportability was not limited to be calculated with high accuracy.

The above-described findings by the present inventors have not been known and have new technical features. Specifically, the present disclosure provides the following aspects.

A parameter determination method according to a first aspect of the present disclosure is a parameter determination method for determining a value of a parameter that is used for a simulation of determining gas or ion transportability in a space inside a pore and that defines a boundary condition at an interface between a wall surface and gas or ions inside the pore, the method including the step of determining the value of a parameter that reproduces a first concentration ratio indicating a ratio of the gas or ion concentration inside the pore to the gas or ion concentration outside the pore as the value of the parameter that defines the boundary condition.

According to the above-described parameter determination method, the value of a parameter that reproduces the first concentration ratio can be determined as the value of the parameter that defines a boundary condition at the interface between the wall surface and gas or ions in the space inside the pore. Consequently, the gas or ion transportability in a space inside the pore can be determined by a simulation in consideration of the aspect of gas or ion adsorption and desorption with respect to the wall surface inside the pore. In this regard, gas or ion transportability refers to gas or ion concentration in a steady state.

Therefore, the parameter determination method according to the first aspect of the present disclosure exerts an effect of enabling the value of the parameter that is used for a simulation technique for determining gas or ion transportability inside a pore with high accuracy to be determined.

The parameter determination method according to a second aspect of the present disclosure may include the step of acquiring the first concentration ratio before the step of determining the value of the parameter in the first aspect.

The parameter determination method according to a third aspect of the present disclosure may include the step of calculating the first concentration ratio before the step of determining the value of the parameter in the first aspect.

In the parameter determination method according to a fourth aspect of the present disclosure, the first concentration ratio may be calculated on the basis of molecular dynamics calculation in the third aspect.

In the parameter determination method according to a fifth aspect of the present disclosure, in the step of determining the value of a parameter, the value of the parameter that is set when a second concentration ratio indicating the ratio of a gas or ion concentration inside the pore determined on the basis of a diffusion equation in which the value of the parameter is applied as the boundary condition to the gas or ion concentration outside the pore is in accord with the first concentration ratio may be determined as the value of the parameter that defines the boundary condition at the interface between the wall surface and the gas or ions in the space inside the pore in any one of the first to fourth steps.

According to the above-described parameter determination method, the value of the parameter can be determined such that the second concentration ratio determined on the basis of the diffusion equation is in accord with the first concentration ratio calculated on the basis of molecular dynamics calculation in the first step. Consequently, the gas or ion transportability inside a pore can be simulated with high accuracy on the basis of a diffusion equation in which the determined value of the parameter is applied as the boundary condition.

Therefore, the gas or ion transportability in a space inside a pore can be determined on the basis of a simple diffusion equation, in which the determined value of the parameter is applied as the boundary condition, without performing molecular dynamics calculation.

In the parameter determination method according to a sixth aspect of the present disclosure, the step of determining the value of a parameter in the fifth aspect may include the step of determining the gas or ion concentration inside the pore and the gas or ion concentration outside the pore by repetitively calculating the diffusion equation in which an arbitrarily set value of the parameter is applied as the boundary condition, the step of deciding whether there is no change in the value of the gas or ion concentration repetitively calculated, the step of deciding whether a second concentration ratio is in accord with the first concentration ratio by comparison, where the concentration ratio indicating the ratio of the gas or ion concentration inside the pore to the gas or ion concentration outside the pore when it is decided that there is no change in the value of the gas or ion concentration is denoted as the second concentration ratio, and the step of determining the value of the parameter that is set when the first concentration ratio is in accord with the second concentration ratio as the value of the parameter that defines the boundary condition at the interface between the wall surface and the gas or ions in the space inside the pore.

In the parameter determination method according to a seventh aspect of the present disclosure, the diameter of the pore may be 10 nm or less in any one of the first to sixth aspects.

In this regard, it is considered that, when the diameter of the pore is as very small as 10 nm or less, an effect of gas adsorption and desorption with respect to the wall surface inside the pore is greater than the effect in the case of a pore having a large diameter. Consequently, the parameter determination method according to the fourth aspect can determine the value of the parameter that defines the boundary condition at the interface between the wall surface and the gas or ions inside the pore, and the gas or ion transportability inside the pore can be determined with high accuracy by a simulation in consideration of the aspect of gas or ion adsorption and desorption with respect to the wall surface inside the pore.

In the parameter determination method according to an eighth aspect of the present disclosure, the pore may contain carbon in any one of the first to seventh aspects.

In the parameter determination method according to a ninth aspect of the present disclosure, the pore may be a pore of a carbon carrier in an electrode catalyst layer in any one of the first to eighth aspects.

In the case in which the pore is an electrode catalyst layer, the gas or ion transportability inside the pore of the electrode catalyst layer can be determined with high accuracy by a simulation in which the determined value of the parameter is applied. Therefore, the power generation performance in the electrode catalyst layer can be predicted.

A simulation method for determining gas or ion transportability inside a pore according to a tenth aspect of the present disclosure includes calculating a change in the gas or ion concentration on the basis of a diffusion equation in which the parameter value determined by a parameter determination method including the step of determining the value of a parameter that reproduces a first concentration ratio indicating the ratio of the gas or ion concentration inside the pore to the gas or ion concentration outside the pore as the value of a parameter that defines the boundary condition.

According to the above-described simulation method for determining gas or ion transportability inside a pore, the value of a parameter that reproduces a first concentration ratio can be determined as the value of a parameter that defines the boundary condition. Consequently, the gas or ion transportability in a space inside the pore can be determined in consideration of the aspect of gas or ion adsorption and desorption with respect to the wall surface inside the pore. In this regard, gas or ion transportability refers to a gas or ion concentration in a steady state.

Therefore, the simulation method for determining gas or ion transportability inside a pore according to a tenth aspect of the present disclosure exerts an effect of enabling the gas or ion transportability inside the pore to be determined with high accuracy.

The embodiment according to the present disclosure will be described below with reference to the drawings. Hereafter, regarding all drawings, the same or corresponding constituent members are indicated by the same references, and explanations thereof may be omitted. In this regard, in the embodiment according to the present disclosure, a method for determining the gas transportability will be described as an example. However, the ion transportability may be determined by the same method.

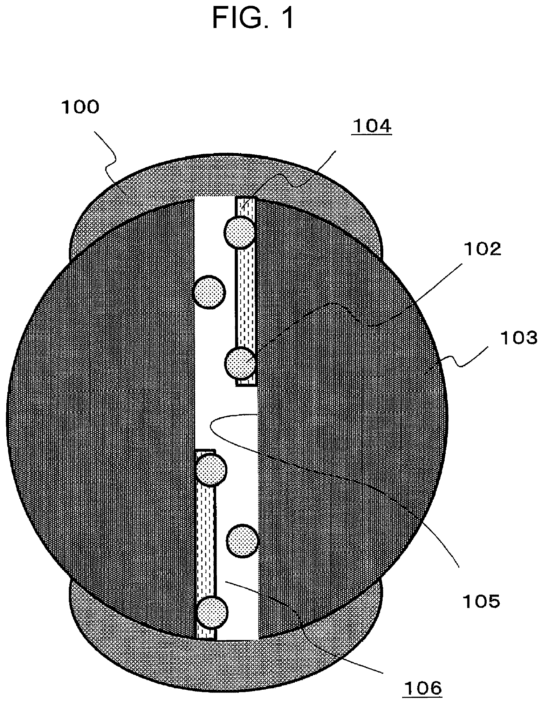

The configuration of a carbon carrier 103 that is the evaluation target of the gas transportability will be described with reference to Fig. 1. Fig. 1 is a schematic diagram showing an example of the carbon carrier 103 having a pore according to the present embodiment. In Fig. 1, one cylindrical carbon pore 105 is formed in the carbon carrier 103 for the sake of facilitating explanation. However, a plurality of pores 105 may be formed, and the shape is not limited to be cylindrical.

As shown in Fig. 1, the carbon carrier 103 according to the embodiment has an outer circumference surface covered with a polymer electrolyte 100 and has inside a nanoscale order carbon pore 105. The carbon carrier 103 can be used as, for example, a cathode-side electrode catalyst layer of a fuel cell.

Metal catalysts 102 are disposed inside the carbon pore 105. In addition, liquid water regions 104 and a gas region 106 are present in the carbon pore 105. In this manner, the carbon carrier 103 has a configuration in which the metal catalysts 102 are included in the carbon pore 105 and the polymer electrolyte 100 is connected to some metal catalysts 102 through the liquid water regions 104.

This configuration of the carbon carrier 103 according to the embodiment can suppress direct contact between the polymer electrolyte 100 and the metal catalysts 102 and can suppress degradation in the activity of the metal catalysts 102.

Regarding the carbon carrier 103 having the above-described configuration, the simulation technique according to the embodiment is intended to reproduce the gas transportability in consideration of gas adsorption and desorption with respect to the wall surface of the carbon pore 105 to calculate the transportability of the gas present in the gas region 106 with high accuracy.

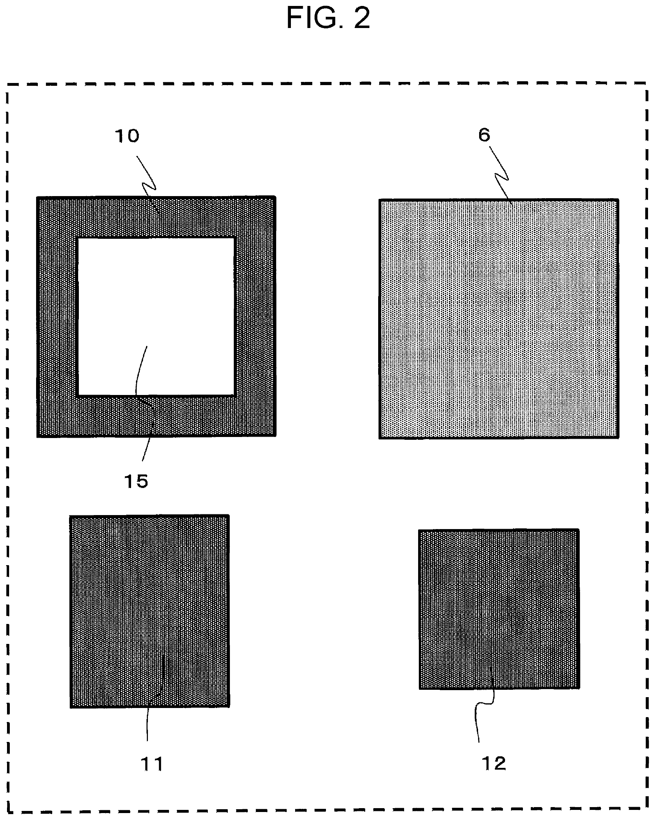

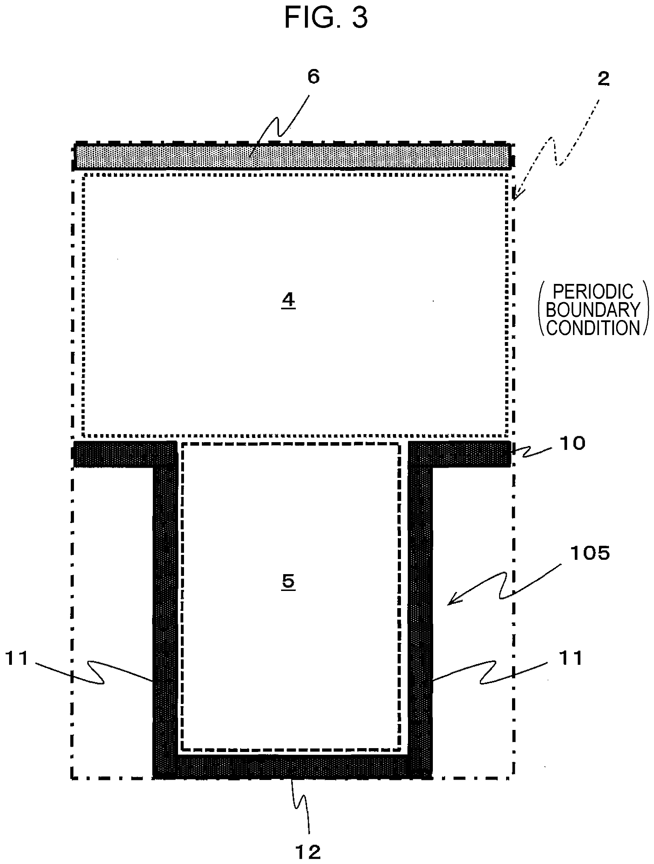

As shown in Figs. 2 and 3, a model simulating a space inside the carbon pore 105 (space inside pore 5) and a space outside the carbon pore 105 (space outside pore 4) is formed. Fig. 2 is a diagram showing an example of each graphite face constituting the model simulating the space inside pore 5 of the carbon pore 105 in the carbon carrier 103 and the space outside pore 4 of the carbon pore 105 according to the embodiment of the present disclosure. Fig. 3 is a diagram showing an example of the model simulating the space inside pore 5 of the carbon pore 105 formed by using the graphite faces shown in Fig. 2 and the space outside pore 4 of the carbon pore 105.

A graphite upper face 10 is a face that is formed of graphite and that has a hole portion 15 with a dimension of, for example, 10 nm $\times$ 10 nm at the center of a face cut into a dimension of, for example, 15 nm $\times$ 15 nm. A graphite side face 11 is a face that is formed of graphite and that is cut into a dimension of, for example, 10 nm $\times$ 12 nm. In the example shown in Fig. 2, the graphite side face 11 has a vertical dimension of 12 nm and a horizontal dimension is 10 nm. A graphite bottom face 12 is a face that is formed of graphite and that is cut into a dimension of, for example, 10 nm $\times$ 10 nm. A repulsion face 6 is a face that is formed of graphite and that is cut into a dimension of, for example, 15 nm $\times$ 15 nm.

The model simulating the space outside pore 4 of the carbon pore 105 and the space inside pore 5 of the carbon pore 105 shown in Fig. 3 is formed by using the graphite upper face 10, the graphite side face 11, the graphite bottom face 12, and the repulsion face 6. Specifically, this model is formed by combining one graphite upper face 10, four graphite side faces 11, one graphite bottom face 12, and one repulsion face 6. That is, as shown in Fig. 3, a space simulating the space inside pore 5 in the carbon pore 105 is formed by using one graphite upper face 10, four graphite side faces 11, and one graphite bottom face 12, and a space simulating the space outside pore 4 of the carbon pore 105 is formed between the repulsion face 6 and the graphite upper face 10.

In the simulation technique according to the present embodiment, on the basis of the resulting model, the concentration ratio indicating the ratio of the gas concentration in the space inside pore 5 to the gas concentration in the space outside pore 4 (first concentration ratio) is determined by molecular dynamics calculation in step (1) below. Subsequently, in step (2) below, the values of parameters (adsorption parameters $A_1$ and $A_2$ described later) to be applied to calculate the gas transportability inside the nanoscale order carbon pore 105 on the basis of a diffusion equation are determined. In step (3) below, the gas transportability inside the carbon pore 105 is calculated on the basis of the diffusion equation to which the values of the parameters determined in step (2) are applied.

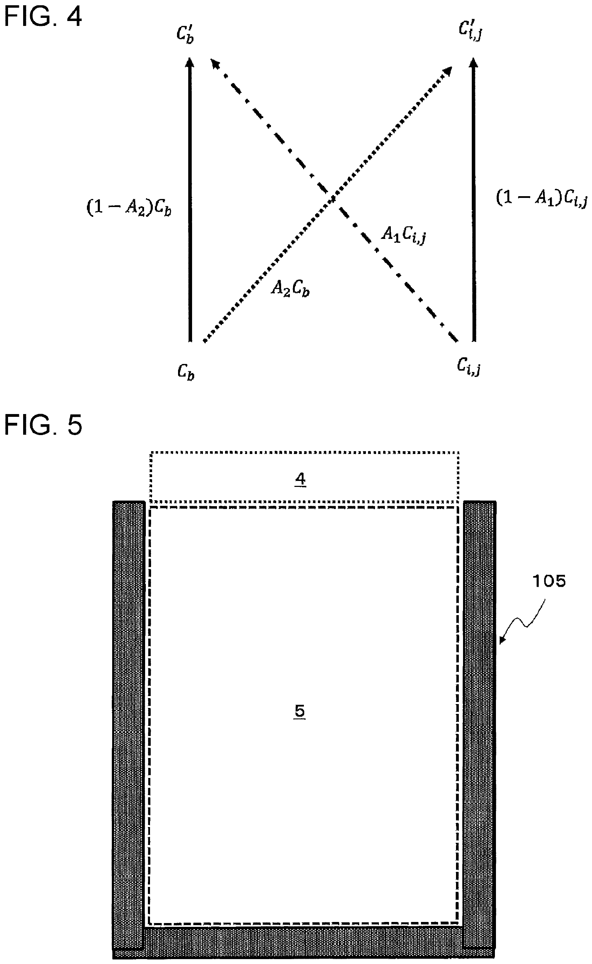

The simulation technique according to the embodiment will be described below with reference to Figs. 4 to 6. In this regard, the simulation technique may be realized by, for example, a simulation apparatus (not shown in the drawing) including a processor (not shown in the drawing) and a memory (not shown in the drawing) reading and performing a program stored in the memory.

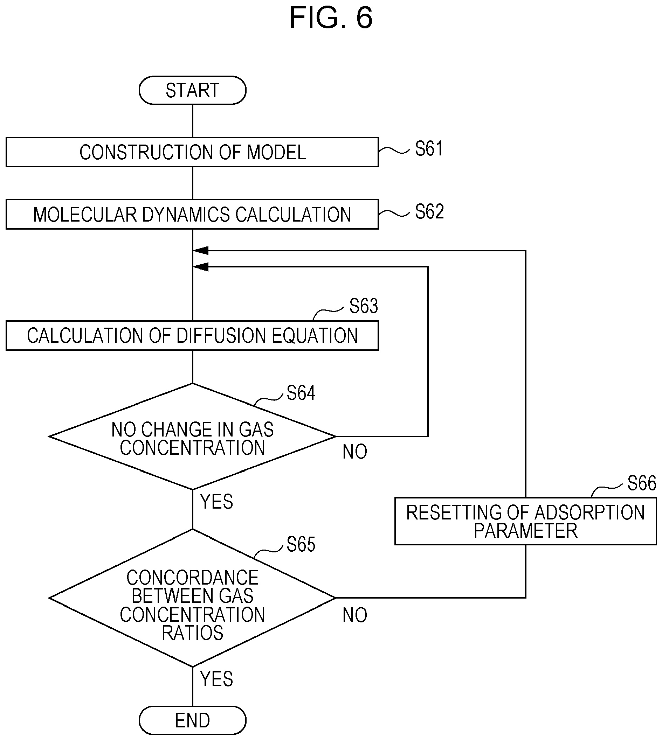

Fig. 4 is a conceptual diagram showing an example of a method for determining an adsorption parameter of oxygen with respect to a wall surface according to the embodiment of the present disclosure. Fig. 4 shows a method for determining the adsorption parameter when oxygen that adsorbs to the wall surface inside the carbon pore 105 is simulated. Regarding Fig. 4, the oxygen concentration in the vicinity of the wall surface of the space inside pore 5 of the carbon pore 105 is denoted as an oxygen concentration $C_{i,j}$, the oxygen concentration on the wall surface of the space inside pore 5 of the carbon pore 105 is denoted as an oxygen concentration $C_b$, the oxygen concentration in the vicinity of the wall surface of the space inside pore 5 of the carbon pore 105 in the calculation step at the next time is denoted as an oxygen concentration $C_{i,j'}$, and the oxygen concentration on the wall surface of the space inside pore 5 of the carbon pore 105 in the calculation step at the next time is denoted as an oxygen concentration $C_b'$. Fig. 5 is a schematic diagram showing an example of a model used to calculate an oxygen concentration according to an embodiment of the present disclosure. Fig. 6 is a flow chart showing an example of a method for determining an adsorption parameter according to an embodiment of the present disclosure.

To calculate the gas transportability inside the carbon pore 105, the contents of the processing of step (1) to step (3) performed by using the simulation technique according to the embodiment are as described below.

In step (1), the ratio of the gas (oxygen) concentration inside the carbon pore 105 to the gas (oxygen) concentration outside the carbon pore 105 is calculated on the basis of the molecular dynamics calculation. In step (2), the value of the adsorption parameter that reproduces the gas concentration ratio calculated in step (1) is determined. In step (3), the gas transportability in the space inside pore 5 is calculated by applying the value of the adsorption parameter determined in step (2) to the interface between the gas and the wall surface of the carbon pore 105.

[Calculation of ratio of gas concentration inside pore to gas concentration outside pore based on molecular dynamics calculation; step (1)]

Step (1) will be described. Step (1) corresponds to "Construction of model" indicated as step S61 and "Molecular dynamics calculation" indicated as step S62 in the flow chart shown in Fig. 6. In this regard, for example, J-OCTA (registered trade mark) known as material physical property analysis software may be used for the molecular dynamics calculation performed in the embodiment.

To begin with, the processing performed in the step of constructing a model (step S61) will be described.

Initially, an appropriate force field is set for each of oxygen molecules and graphite. Any force field may be set, but it is favorable to use a force field with high reliability. A generic force field (for example, AMBER, DREIDING, and OPLIS for organic molecules and SPCE for water molecules), a literature value, or the like may be adopted as the force field with high reliability. It is desirable that the validity of the force field to be set be evaluated by performing calculation for examining the reproducibility of physical property values, for example, density and diffusion coefficient, in an actual system.

Regarding the molecular dynamics calculation, interaction between oxygen molecules, in which energy of an oxygen molecule is changed in accordance with the intermolecular distance, is calculated on the basis of the force field set as described above. A change in the coordinates of the oxygen molecule subjected to the interaction is calculated. This calculation is repeated with time development so as to obtain data indicating changes with time in the coordinates of the oxygen molecule.

Subsequently, graphite faces shown in Fig. 2 are prepared, and these are assembled to form a model simulating the space outside pore 4 and the space inside pore 5 of the carbon pore 105 shown in Fig. 3. As described above, the carbon pore 105 may be formed by using one graphite upper face 10, four graphite side faces 11, and one graphite bottom face 12.

Specifically, the graphite side faces 11 are arranged so as to become perpendicular to the graphite upper face 10 arranged horizontally. One side (side having a dimension of 10 nm) of each of the graphite side faces 11 is arranged so as to be brought into contact with a corresponding side of the hole portion 15 of the graphite upper face 10. At the end portion opposite to the graphite upper face 10 of the tube portion formed by combining the four graphite side faces 11, as described above, the graphite bottom face 12 is arranged in the horizontal direction so as to be brought into perpendicular contact with the graphite side faces 11 and to block the opening of the tube portion. In this manner, the model simulating the carbon pore 105 is formed. Further, the repulsion face 6 is arranged horizontally at the position 12 nm apart from and above the graphite upper face 10. In the above-described procedure, the model simulating the space outside the carbon pore 105 and the carbon pore 105 shown in Fig. 3 is formed.

In next step S62, the model formed in step S61 is used, and each of the average number of oxygen molecules present in the space outside pore 4 and the average number of oxygen molecules present in the space inside pore 5 is calculated on the basis of the molecular dynamics calculation.

Specifically, the interaction between an oxygen molecule and the repulsion face 6 is set to be a Lennard-Jones potential type non-bonding interaction. Regarding the Lennard-Jones potential type non-bonding interaction set here, the value of the potential depth $\varepsilon$ [kcal/mol] is set to be 0.0001, the cut-off distance is set to be 3 Å, and the interaction between the repulsion face 6 and an oxygen atom is set to be a weak repulsive force only. Setting the interaction between the repulsion face 6 and an oxygen atom to be a weak repulsive force only enables the oxygen atom to be suppressed from adsorbing to the repulsion face 6. Further, a periodic boundary condition is applied to the analysis cell 2. Consequently, a bulk gas phase state can be simulated in the space outside pore 4 of the carbon pore 105.

Thereafter, the temperature is set at T = 353 [K], oxygen molecules at a density corresponding to 1 atmosphere are randomly inserted into the space outside pore 4, the NVT ensemble is subjected to time development, and relaxation calculation is performed until the energy becomes unchanged with time.

Further, to obtain a satisfactory time average, additional time development is performed for several ns, and the trajectories of oxygen molecules are acquired. The average number of oxygen molecules present in the space outside pore 4 and the average number of oxygen molecules present in the space inside pore 5 are calculated from the resulting trajectories and are converted to the oxygen concentration in the space outside pore 4 and the oxygen concentration in the space inside pore 5, respectively, by being divided by the respective space volumes. Subsequently, the oxygen concentration ratio determined by "(oxygen concentration in space inside pore 5)/(oxygen concentration in space outside pore 4)" is taken as the gas concentration ratio (first concentration ratio). A gas concentration ratio more than 1 indicates that gas (oxygen) is adsorbed in the space inside pore 5.

In the above-described procedure, the ratio of the gas concentration inside the pore to the gas concentration outside the pore is calculated on the basis of the molecular dynamics calculation.

[Determination of adsorption parameter that reproduces gas concentration ratio; step (2)]

Next, processing for determining adsorption parameters (parameters) $A_1$ and $A_2$ capable of reproducing the gas concentration ratio calculated on the basis of the above-described molecular dynamics calculation will be described. In the processing, the adsorption parameters $A_1$ and $A_2$ may be determined while a mesh (cell) is set by using, for example, the technique of the finite element method. The main purpose of step (2) is to calculate the adsorption parameters $A_1$ and $A_2$ capable of reproducing the gas concentration ratio calculated on the basis of the molecular dynamics calculation in step (1). Meanwhile, step (2) corresponds to calculation of diffusion equation (step S63), branch processing to decide whether there is no change in gas concentration (step S64), branch processing to decide whether there is concordance between gas concentration ratios (step S65), and resetting of adsorption parameter (step S66) in the flow chart shown in Fig. 6.

The adsorption parameters $A_1$ and $A_2$ to be determined will be described. As shown in Fig. 4, when the oxygen concentration of a mesh in contact with the wall surface is denoted as the oxygen concentration $C_{i,j}$ in the vicinity of the wall surface, it is possible to conjecture that gas adsorption to the wall surface is the state in which the oxygen concentration $C_{i,j}$ in the vicinity of the wall surface and the oxygen concentration $C_b$ on the wall surface exchange part of the concentration with each other so as to change with time and reach equilibrium. In this regard, "exchange part of the concentration with each other" denotes that an ensemble of oxygen molecules present in the vicinity of the wall surface and an ensemble of oxygen molecules present on the wall surface exchange the same proportion of oxygen molecules with each other. That is, in Fig. 4, the boundary condition is set such that a mesh portion on the wall surface (wall surface portion) and a mesh portion adjacent thereto (portion in the vicinity of the wall surface) exchange the same proportion of oxygen molecules with each other.

The adsorption parameters $A_1$ and $A_2$ are introduced. The oxygen concentration $C_{i,j}$ in the vicinity of the wall surface releases $A_1 \times C_{i,j}$ that is $A_1$ times the concentration of itself to the oxygen concentration $C_b{}'$ on the wall surface at the next time. The residue $(1 - A_1) \times C_{i,j}$ remains in the oxygen concentration $C_{i,j}{}'$ in the vicinity of the wall surface at the next time. Meanwhile, oxygen concentration $C_b$ on the wall surface releases $A_2 \times C_b$ that is $A_2$ times the concentration of itself to the oxygen concentration $C_{i,j}{}'$ in the vicinity of the wall surface at the next time. The residue $(1 - A_2) \times C_b$ remains in the oxygen concentration $C_b{}'$ on the wall surface at the next time. Therefore, each of the oxygen concentration $C_{i,j}{}'$ in the vicinity of the wall surface at the next time and the oxygen concentration $C_b{}'$ on the wall surface at the next time can be represented by mathematical formula (1) below. In mathematical formula (1), each of $A_1$ and $A_2$ [-] represents an adsorption parameter, $C_{i,j}$ [mol/m$^3$] represents an oxygen concentration in the vicinity of the wall surface, $C_b$ [mol/m$^3$] represents an oxygen concentration on the wall surface, $C_{i,j}{}'$ [mol/m$^3$] represents an oxygen concentration in the vicinity of the wall surface at the next time, and $C_b{}'$ [mol/m$^3$] represents an oxygen concentration on the wall surface at the next time.

[Math. 1]

$$C'_{i,j} = (1 - A_1)C_{i,j} + A_2 C_b$$
$$C'_b = A_1 C_{i,j} + (1 - A_2)C_b \qquad \cdots (1)$$

To determine the above-described adsorption parameters $A_1$ and $A_2$, calculation of diffusion equation (step S63) is performed for the purpose of calculating the oxygen concentration in the space inside pore 5 of the carbon pore 105. Regarding the diffusion equation, mathematical formula (2) above is applied as the boundary condition at the interface between the oxygen and the carbon pore 105, and mathematical formula (2) below is computed by using the model shown in Fig. 5 (oxygen concentration calculation model). That is, the gas behavior in the space inside pore 5 can be expressed by Laplace equation represented by mathematical formula (2). In mathematical formula (2), C [mol/m$^3$] represents an oxygen concentration.
[Math. 2]

$$\nabla^2 C = 0 \qquad \cdots (2)$$

Specifically, as an initial value, an arbitrary oxygen concentration, for example, 1 [mol/m$^3$], is set in the space outside pore 4. Regarding other regions, an arbitrary value, for example, 0 [mol/m$^3$], is set. In this regard, mathematical formula (1) above is applied as the boundary condition at the interface of the carbon pore 105, and calculation is performed such that the oxygen fed from the space outside pore 4 to the space inside pore 5 satisfies the diffusion legal formula represented by mathematical formula (2) above.

Regarding this calculation, immediately after start of the calculation, each of the space outside pore 4 and the space inside pore 5 has an oxygen concentration distribution in accordance with the initial value. However, when the calculation is repeated, convergence to the oxygen concentration based on the initial value applied to the space outside pore 4 and the boundary condition represented by mathematical formula (1) proceeds. In this regard, each of the oxygen concentration in the space outside pore 4 and the oxygen concentration in the space inside pore 5 refers to an averaged oxygen concentration in each space.

After the calculation of the diffusion equation is performed, the decision processing shown in the following step S64 is performed. This decision processing is branch processing to decide whether there is no change in the oxygen concentration in the space inside pore 5. In repetitive calculation of the diffusion equation represented by mathematical formula (1), the temporary solution obtained last time is compared with the solution obtained this time, and when the amount of change in the value is less than the threshold value, it is decided that the gas concentration is not changed ("YES" in step S64). If decision is "YES" in step S64, it is assumed that the oxygen concentration has reached the steady state, and shift to the following step S65 is performed. Meanwhile, the temporary solution obtained last time is compared with the solution obtained this time, and when the amount of change in the value is more than or equal to the threshold value, it is decided that the gas concentration is changed ("NO" in step S64). If it is decided that the gas concentration is changed, return to step S63 that is a step of calculating the diffusion equation is performed, and additional repetitive calculation is performed.

In the case in which it is ascertained that steady state is reached and step S65 is selected, the gas concentration ratio (second concentration ratio) is calculated by dividing the oxygen concentration set in the space outside pore 4 by the average oxygen concentration in the space inside pore 5. The branch processing of step S65 decides whether the gas concentration ratio (first concentration ratio) calculated on the basis of the molecular dynamics calculation (step S62) in step (1) above is in accord with the gas concentration ratio calculated on the basis of the calculation of diffusion equation (step S63) in step (2) above. In step S65, if the simulation device according to the present embodiment decides that there is no concordance ("NO" in step S65), the values of the adsorption parameters $A_1$ and $A_2$ are reset (step S66). Subsequently, return to step S63 is performed, and the diffusion equation is calculated on the basis of the reset values of the adsorption parameters $A_1$ and $A_2$.

In step S65, if the simulation device according to the present embodiment decides that there is concordance, the adsorption parameters $A_1$ and $A_2$ that lead to the gas concentration ratio calculated on the basis of the molecular dynamics calculation in step (1) can be determined. In other words, the adsorption parameters $A_1$ and $A_2$ capable of reproducing gas adsorption to the wall surface of the carbon pore 105 can be determined. Therefore, processing of step (2) is finished.

In the method for determining the adsorption parameter by using the simulation device according to the present disclosure, step (1) above is performed, the gas concentration ratio determined in step (1) is compared with the gas concentration ratio calculated by calculation of the diffusion equation (step S63) in step (2), and it is decided whether there is concordance. However, step (1) is not limited to be performed in the method for determining the adsorption parameter according to the embodiment of the present disclosure. For example, the ratio of the gas concentration in the space inside pore 5 to the gas concentration in the space outside pore 4 may be determined in advance by another simulation device, and, in step (2), the gas concentration ratio determined in advance may be acquired so as to compare the acquired gas concentration ratio and the gas concentration ratio calculated by calculation of the diffusion equation.

[Calculation of gas transportability in space inside pore by applying adsorption parameter to interface between gas and wall surface of carbon pore; step (3)]

Next, step (3) will be described. In step (3), mathematical formula (1) into which the adsorption parameters $A_1$ and $A_2$ obtained in step (2) have been substituted is applied as the boundary condition on the wall surface of the carbon pore 105. Then, the gas transportability in consideration of the effect of gas adsorption to the wall surface of the carbon pore 105 can be calculated by solving the diffusion equation represented by mathematical formula (2) of the gas in the space inside pore 5.

As described above, in step (3), the gas transportability in the space inside pore 5, that is, the gas concentration in the steady state, can be determined with high accuracy by simulation using the diffusion equation in which the adsorption parameters $A_1$ and $A_2$ obtained in step (2) according to the embodiment of the present disclosure are applied as the boundary condition at the interface between the wall surface of the carbon pore 105 and the gas.

Consequently, in the case in which the carbon carrier 103 according to the embodiment is used for, for example, an electrode catalyst layer of a fuel cell, the optimum structure of the fuel cell catalyst layer and the production process thereof can be determined without trial production. Therefore, improvement of the performance, cost reduction, and development time reduction of the fuel cell catalyst layer can be realized.

In the description of the embodiment according to the present disclosure, oxygen is adopted as an example of the gas that moves in the space inside pore 5. However, the gas is not limited to oxygen, and a gas other than oxygen may be adopted.

Meanwhile, in the method for determining an adsorption parameter according to the embodiment of the present disclosure, the gas transportability inside the carbon pore 105 is calculated. However, the method can be applied to not only the gas transportability but also ion transportability. For example, in a lithium ion battery, lithium ions move through a negative electrode having a layered carbon structure. Therefore, the lithium ion transportability in the negative electrode of the lithium ion battery can be simulated by using the method for determining an adsorption parameter according to the embodiment of the present disclosure.

Industrial Applicability

The present disclosure can be widely applied when gas or ion transportability in a space inside a pore is determined by a simulation technique. Reference Signs List

2      analysis cell
4      space outside pore

5      space inside pore
6      repulsion face
10      graphite upper face
11      graphite side face
12      graphite bottom face
15      hole portion
100      polymer electrolyte
102      metal catalyst
103      carbon carrier
104      liquid water region
105      carbon pore
106      gas region
$A_1$      adsorption parameter
$A_2$      adsorption parameter

**Claims**

1. A parameter determination method for determining a value of a parameter that is used for a simulation of determining gas or ion transportability in a space inside a pore and that defines a boundary condition at an interface between a wall surface and gas or ions inside the pore, the method comprising: determining the value of a parameter that reproduces a first concentration ratio indicating a ratio of the gas or ion concentration inside the pore to the gas or ion concentration outside the pore as the value of the parameter that defines the boundary condition.

2. The parameter determination method according to Claim 1 comprising acquiring the first concentration ratio before the determining of the value of the parameter.

3. The parameter determination method according to Claim 1 comprising calculating the first concentration ratio before the determining of the value of the parameter.

4. The parameter determination method according to Claim 3 wherein the first concentration ratio is calculated on the basis of molecular dynamics calculation.

5. The parameter determination method according to any one of Claims 1 to 4, wherein in the determining of the value of a parameter, the value of the parameter that is set when a second concentration ratio indicating the ratio of the gas or ion concentration inside the pore determined on the basis of a diffusion equation in which the value of the parameter is applied as the boundary condition to the gas or ion concentration outside the pore is in accord with the first concentration ratio is determined as the value of the parameter that defines the boundary condition at the interface between the wall surface and the gas or ions in the space inside the pore.

6. The parameter determination method according to Claim 5, wherein the determining of the value of a parameter comprises:

    determining the gas or ion concentration inside the pore and the gas or ion concentration outside the pore by repetitively calculating the diffusion equation in which an arbitrarily set value of the parameter is applied as the boundary condition, deciding whether there is no change in the value of the gas or ion concentration repetitively calculated,
    deciding whether a second concentration ratio is in accord with the first concentration ratio by comparison, where the concentration ratio indicating the ratio of the gas or ion concentration inside the pore to the gas or ion concentration outside the pore when it is decided that there is no change in the value of the gas or ion concentration is denoted as the second concentration ratio, and
    determining the value of the parameter that is set when the first concentration ratio is in accord with the second concentration ratio as the value of the parameter that defines the boundary condition at the interface between the wall surface and the gas or ions in the space inside the pore.

7. The parameter determination method according to any one of Claims 1 to 6, wherein the diameter of the pore is 10 nm or less.

8. The parameter determination method according to any one of Claims 1 to 7, wherein the pore contains carbon.

9. The parameter determination method according to any one of Claims 1 to 8, wherein the pore is a pore of a carbon carrier in an electrode catalyst layer.

10. A simulation method for determining gas or ion transportability inside a pore, comprising calculating a change in the gas or ion concentration on the basis of a diffusion equation in which the parameter value determined by the parameter determination method according to any one of Claims 1 to 9 is applied as the value of a parameter that defines the boundary condition at the interface between the wall surface and the gas or ions inside the pore.

# FIG. 1

# FIG. 2

# FIG. 3

FIG. 4

$$C_b'$$

$$C_{i,j}'$$

$$(1-A_2)C_b$$

$$(1-A_1)C_{i,j}$$

$$A_1C_{i,j}$$

$$A_2C_b$$

$$C_b$$

$$C_{i,j}$$

FIG. 5

# FIG. 6

START

CONSTRUCTION OF MODEL — S61

MOLECULAR DYNAMICS CALCULATION — S62

CALCULATION OF DIFFUSION EQUATION — S63

NO CHANGE IN GAS CONCENTRATION — S64

NO

YES

CONCORDANCE BETWEEN GAS CONCENTRATION RATIOS — S65

NO

RESETTING OF ADSORPTION PARAMETER — S66

YES

END

## INTERNATIONAL SEARCH REPORT

International application No.

PCT/JP2019/011625

### A. CLASSIFICATION OF SUBJECT MATTER

Int. Cl. H01M4/88(2006.01)i, B01J35/10(2006.01)i, C01B32/00(2017.01)i,
G06F17/50(2006.01)i, G16Z99/00(2019.01)i, H01M4/86(2006.01)i,
H01M4/96(2006.01)i, H01M8/10(2016.01)n
According to International Patent Classification (IPC) or to both national classification and IPC

### B. FIELDS SEARCHED

Minimum documentation searched (classification system followed by classification symbols)
Int. Cl. H01M4/88, B01J35/10, C01B32/00, G06F17/50, G16Z99/00, H01M4/86,
H01M4/96, H01M8/10

Documentation searched other than minimum documentation to the extent that such documents are included in the fields searched
Published examined utility model applications of Japan        1922-1996
Published unexamined utility model applications of Japan      1971-2019
Registered utility model specifications of Japan              1996-2019
Published registered utility model applications of Japan      1994-2019

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 7-256112 A (WATANABE, Masahiro) 09 October 1995, paragraph [0009] & US 6168772 B1, column 2, line 52 to column 3, line 9 | 1-10 |
| A | JP 2004-33892 A (TOYOBO CO., LTD.) 05 February 2004, paragraphs [0019], [0020] (Family: none) | 1-10 |
| A | JP 2005-294107 A (TOYOTA MOTOR CORP.) 20 October 2005, paragraphs [0041]-[0044] (Family: none) | 1-10 |
| A | JP 2003-201417 A (N.E. CHEMCAT CORP.) 18 July 2003, paragraph [0010] & US 2003/0108481 A1, paragraph [0012] | 1-10 |

☒ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 03.06.2019 | 11.06.2019 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

## INTERNATIONAL SEARCH REPORT

International application No.

PCT/JP2019/011625

C (Continuation). DOCUMENTS CONSIDERED TO BE RELEVANT

| Category* | Citation of document, with indication, where appropriate, of the relevant passages | Relevant to claim No. |
|---|---|---|
| A | WO 2014/175097 A1 (NISSAN MOTOR CO., LTD.) 30 October 2014, paragraphs [0011]-[0016], [0029], fig. 2 & US 2016/0072134 A1, paragraphs [0017]-[0025], [0038], fig. 2 | 1-10 |
| A | JP 2010-153354 A (TOYOTA MOTOR CORP.) 08 July 2010, paragraphs [0032], [0034], fig. 1-3 (Family: none) | 1-10 |
| P, A | JP 2018-181838 A (PANASONIC INTELLECTUAL PROPERTY MANAGEMENT CO., LTD.) 15 November 2018, paragraph [0067], fig. 4, 5 & EP 3392938 A1, paragraphs [0064], [0065], fig. 4, 5 | 1-10 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. MUZAFFAR ; T. KADYK ; M. EIKERLING.** Physical Modeling of the Proton Density in Nanopores of PEM Fuel Cell Catalyst Layers. *Electrochimica Acta,* 2017, vol. 245, 1048-1058